Europäisches Patentamt

**European Patent Office**

**Office européen des brevets**

(19)

(11) Publication number: **0 108 661**
**A1**

(12)

# EUROPEAN PATENT APPLICATION

(21) Application number: **83401912.7**

(22) Date of filing: **29.09.83**

(51) Int. Cl.³: **A 61 L 2/08**
**A 61 F 9/00, A 61 K 9/06**

(30) Priority: **04.10.82 US 432574**

(43) Date of publication of application:
**16.05.84 Bulletin 84/20**

(84) Designated Contracting States:
**DE FR GB IT NL SE**

(71) Applicant: MERCK & CO. INC.
126, East Lincoln Avenue P.O. Box 2000
Rahway New Jersey 07065(US)

(72) Inventor: Harwood, Richard J.
3219 Adams Court North
Bensalem Pennsylvania 19020(US)

(74) Representative: Warcoin, Jacques et al,
Cabinet Régimbeau 26, avenue Kléber
F-75116 Paris(FR)

(54) Stabilized hydroxypropyl cellulose ophthalmic inserts and process to sterilize the same.

(57) The invention deals with an opthalmic insert sterilized by means of gamma radiation comprising hydroxypropyl cellulose and from 0 to 35 % by weight medicament which at the time of irradiation consists essentially of a water content of from 6 to 12 % by weight and with method for sterilizing an ophthalmic insert.

EP 0 108 661 A1

0108661

- 1 -                          16734

TITLE OF THE INVENTION
STABILIZED HYDROXYPROPYL CELLULOSE
OPHTHALMIC INSERTS AND PROCESS TO STERILIZE THE SAME

BACKGROUND OF THE INVENTION

This invention relates to stabilized hydroxypropyl cellulose ophthalmic inserts. It further relates to methods of sterilizing ophthalmic inserts comprising hydroxypropyl cellulose as well as two methods of packaging such inserts.

Hydroxypropyl cellulose was previously proposed as a ophthalmic insert material for the eye. For example, U.S.S.N. 166,423, filed July 7, 1980, teaches the use of such inserts for carrying a medicament which has a therapeutic or prophylactic effect in the eye. More recently hydroxypropyl cellulose was found to have itself a therapeutic usefulness in treating the disease of the eye known as dry-eye syndrome; U.S. 4,343,787. Since ophthalmic inserts as well as any material being used to treat the eye must be sterile, it follows that some means to sterilize ophthalmic inserts must be employed. Since the use of a bacteriostatic or a bactericidal agent in ophthalmic products is becoming more and more regarded with disfavor, other methods of preparing the inserts such as manufacture from sterile materials under sterile conditions were considered. Preparation by use of sterile materials under sterile conditions is not only time-consuming, but is expensive and difficult to control. Clearly, exposure to gamma radiation is the superior means of sterilizing ophthalmic inserts.

Unfortunately, exposure to gamma radiation was found to cause some not unsubstantial degree of cross-linking of the hydroxypropyl cellulose. This cross-linking occurred on a random basis, and random cross-linking may be radiation dose dependent. That is to say, those inserts exposed to maximal radiation were highly cross-linked, and those inserts only minimally exposed to a sterilizing dose of radiation were similarly minimally cross-linked.

Thus a production run of ophthalmic inserts, depending upon the location of the package relative to the radiation source, would have ophthalmic inserts that have a variable dissolution rate in the eye. This variable dissolution rate would naturally lead to a highly undesirable variable introduced into the therapeutic product. The amount of dosage released in a given time would thus vary from insert to insert. This is highly undesirable since it is extremely important that each patient receive a reproducable delivery rate of medicament from insertion to insertion.

Hydroxypropyl cellulose is a non-ionic water soluble cellulose ether having properties which are uniquely suitable for use in the preparation of ophthalmic inserts. Thus, it is water soluble and hence dissolves in the aqueous lacrimal fluids. In addition, it is thermoplastic and can therefore be advantageously combined with the ophthalmic drug using conventional plastic processing procedures such as compression molding, injection molding and extrusion prior to unit dose subdivision. Hydroxypropyl cellulose is available in several polymeric forms, all of which are suitable in the preparation of the ophthalmic inserts of the present invention. Thus, the products sold by Hercules Incorporated of Wilmington, Delaware under the name KLUCEL such as KLUCEL HF, HWF, MF, GF, JF, LF, and EF which are intended for food or pharmaceutical use are particularly useful in preparing the new inserts of our invention.

The compositions of this invention can be prepared by various methods. Thus, the drug and the hydroxypropyl cellulose can be dissolved in a suitable solvent and the solution evaporated to afford a thin film comprising the hydroxypropyl cellulose and the drug which can then be subdivided to prepare suitable inserts containing the desired amount of the medicament. Alternatively, and in accordance with a preferred embodiment of our invention, we find that the inserts can be prepared most conveniently using the thermoplastic properties of the hydroxypropyl cellulose. For example, the medicament and the hydroxpropyl cellulose can be warmed together at temperatures between about 65°C

and 205°C and the resulting mixture molded to form a thin film. It is generally preferred to prepare the inserts by molding or extrusion in accordance with procedures which are well known in the art. The molded or extruded product can then be subdivided to afford inserts of suitable size for administration in the eye. For example, castings or compression molded films having a thickness of about 0.5 mm to 1.5 mm can be subdivided to obtain suitable inserts in the form of squares, rectangles, circles, semi-circles, and the like containing the desired amount of active ingredient. Rectangular segments of the cast or compressed film having a thickness between about 0.5 and 1.5 mm can be cut to afford shapes such as rectangular plates of 4 x 5-15 mm or ovals of comparable size. Similarly, extruded rods having a diameter between about 0.5 and 1.5 mm can be cut into suitable sections to provide the desired dosage of medicament. For example, rods of 1.0 to 1 5 mm in diameter and about 10 mm long are found to be satisfactory. The inserts may also be directly formed by injection molding. All of the ophthalmic inserts prepared in accordance with the present invention should be formed so that they do not have any sharp edges or corners which could cause damage to the eye.

The ocular inserts prepared in accordance with this invention can also contain plasticizers to make the ophthalmic inserts more pliable. Plasticizers suitable for this purpose must, of course, also be completely soluble in the lacrimal fluids of the eye. Examples of suitable plasticizers that might be mentioned are water, polyethylene glycol, propylene glycol, glycerine, trimethylol
propane, di and tripropylene glycol, hydroxypropyl
sucrose and the like. Typically, such plasticizers
can be present in the ophthalmic insert in an amount
ranging from about 0% to about 30% by weight. A
particularly preferred plasticizer is water which is
present in amounts of at least about 6% and more
preferably at least about 10%. In actual practice, a
water content of from about 6% to about 12% is
preferred since it may be easily accomplished and
adds the desired softness and pliability to the
insert.

When plasticizing the solid medicinal
product with water, the medicinal product is
contacted with air having a relative humidity of at
least about 40% until said product picks up at least
about 6% water and becomes softer and more pliable.
In a preferred embodiment, the relative humidity of
the air is from about 60% to about 99% and the
contacting is continued until the water is present in
the product in amounts of from about 6% to about 12%.

Suitable drugs which can be administered by
the inserts of the present invention that might be
mentioned are antibacterial substances such as
ß-lactam antibiotics, tetracyclines, chloramphenicol,
neomycin, gramicidin, bacitracin, sulfonamides;
aminoglycoside antibiotics such as gentamycin,
kanamycin, amikacin, sisomicin; and tobramycin;
nitrofurazones, and the like; antihistaminics and
decongestants such as pyrilamine, chlorpheniramine,
tetrahydrazoline, antazoline, and the like; antiinflammatories such as cortisone, hydrocortisone,
betamethasone, dexamethasone, fluocortolone, prednisolone, triamcinolone, indomethacin, Clinoril, its salts and its corresponding sulfide, and the like; miotics and anticholinesterases such as echothiophate, pilocarpine, physostigmine salicylate, diisopropylfluorophosphate, and the like; mydriatics such as atropine, homatropine, scopolamine, hydroxyamphetamine, and the like; and other medicaments used in the treatment of eye 3-morpholino-4-(3-$\underline{t}$-butylamino-2-hydroxypropoxy)-1,2,5-thiadiazole, i.e., Timolol especially as the hydrogen maleate salt.

These drugs or derivatives thereof such as salts, covalent derivatives, for example, esters or amides, or other therapeutically active forms can be admixed with the hydroxypropyl cellulose in the form of the water soluble inserts prepared as described above for the treatment of various eye diseases. These forms are especially advantageous for the treatment of conditions where prolonged drug administration is indicated, for example, in eye diseases or eye disorders such as uveitis glaucoma, diseases of the cornea such as, for example, purulent keratitis, herpes simplex keratitis, herpes zoster, acne rosacea, interstitial keratitis, and the like, diseases of the orbit such as exophthalmos and periostitis and diseases of the conjunctiva such as mucopurulent conjunctivitis and ophthalmia. Also, this method of the administration of ophthalmic active drugs can be used when post-operative treatment is needed after retinal or cataract surgery.

The method of administering drugs as a water soluble insert is especially useful in the administration of pilocarpine to treat glaucoma, a condition characterized by an increase in intraocular pressure.  One treatment of this condition involves the use of solutions of pilocarpine acid salts which are administered to the eye in the form of drops at frequent intervals.  By inserting the ophthalmic inserts of the present invention, a miotic response of up to 9-10 hours may be obtained in rabbits compared to the short response of 2-4 hours obtained by the administration of drops.  The pilocarpine can be administered preferably in the form of acid salts of this drug such as the hydrochloride, alginate, pamoate, and the like.  The ophthalmic inserts of the present invention can contain up to about 35% by weight of pilocarpine to afford a dose of about 1-6 mgs of pilocarpine per insert.

In general, the ophthalmic inserts of the present invention will contain from about 0.1 to about 35% of the medicament, from about 65 to about 99.9% of hydroxypropyl cellulose and from 0 to about 30% of plasticizer, preferably 6-12% water.  In special situations, however, the amounts may be varied to increase or decrease the dosage schedule.  If desired, the insert may also contain in addition to the plasticizers, buffering agents and preservatives.  Suitable water soluble preservatives which may be employed in the insert are sodium bisulfite, sodium thiosulfate, ascorbate, benzalkonium chloride, chlorobutanol, thimerosal, phenylmercuric borate, parabens, benzyl alcohol and phenylethanol.  These agents may be present in amounts of from 0.001 to 5% by weight of solid insert, and preferably 0.1 to 2%.  Suitable water soluble buffering agents are alkali, alkali earth carbonates, phosphates, bicarbonates, citrates, borates, and the like, such as sodium phosphate, citrate, borate, acetate, bicarbonate and carbonate. These agents may be present in amounts sufficient to obtain a pH of the system of between 5.5 to 8.0 and especially 7-8; usually up to about 2% by weight of polymer.

The inserts as is well known, can be prepared to dissolve in any given length of time, and accordingly the drug as well, by merely adjusting the size and weight of the insert, the molecular weight of the polymer and/or by the use of other agents such as plactizers. Typically for an insert which contains approximately 10% by weight of water and about 15-35% by weight of drug such as indomethacin and pilocarpine pamoate, and is from about 8-12 mg in weight. The dissolution rate is from about 0.5 mg/hour to about 3 mg/hour. Suitably, however, the dissolution rate may be from about 0.004 mg/hour up to about 20 mg/hour but preferably is from about 0.04 mg/hour to about 4.0 mg/hour and especially from about 0.08 mg/hour to about 3.0 mg/hour.

The detailed examples describing specific methods of preparing the ophthalmic inserts incorporating hydroxypropyl cellulose included in Serial No. 166,423 filed July 7, 1980, which examples thereof are incorporated herein by reference.

Thus, it was highly desirable that some means be found to protect the inserts from cross-linking. We have now found that ophthalmic inserts comprising hydroxypropyl cellulose can be sterilized by exposure to gamma radiation if they are made to contain or, in the alternative, are surrounded by a specified moisture content. Thus, if ophthalmic inserts are allowed to reach equilibrium with a relative humidity of between 60 and 80% at room temperature (20°C) so that the inserts have an equilibrium moisture content of not less than 6 nor more than 12% a reproducible insert will be obtained when exposed to gamma radiation to achieve sterilization. The prior literature suggests that exposure to gamma radiation sufficient to achieve sterilization of hydroxypropyl cellulose will also result in such a degree of cross-linking and chain scission that the material is no longer suitable as a biodegradable ophthalmic insert. U.S. Patent 3,779,706 employs radiation through high energy electron beam sterilization and such is to be contrasted with gamma irradiation.

## Example 1

| | |
|---|---|
| Hydroxypropyl Cellulose inserts | 5 mg.** |
| Water | 6% w/w |

*Klucel HF - Hercules, Inc.
**Can range from 1 mg. to 100 mg.

The hydroxypropyl cellulose inserts are exposed to 60% relative humidity at room temperature until equilibrium moisture content is reached -- approximately 6% on a weight basis.

Place the hydrated inserts into a tightly closed package. Expose the packaged, hydrated inserts to gamma radiation. In-vitro dissolution data of these inserts is shown below.

| Radiation Dose | Percent Hydroxypropyl Cellulose Dissolved | | | |
|---|---|---|---|---|
| Range | 2 hr. | 4 hr. | 7 hr. | 23 hr. |
| 0 Mrad | 22 | 48 | 77 | 102 |
| 0.7 to 1.0 Mrad | 19 | 44 | 75 | 99 |
| 1.4 to 1.7 Mrad | 14 | 32 | 66 | 101 |
| 2.2 to 2.5 Mrad | 13 | 22 | 36 | 100 |

## Example 2

Rx

|  |  |
|---|---|
| Hydroxypropyl Cellulose* inserts | 5 mg.** |
| Water | 9.0% w/w |

*Klucel HF - Hercules, Inc.
**Can range from 1 mg. to 100 mg.

The hydroxypropyl cellulose inserts are exposed to 80% relative humidity at room temperature until equilibrium moisture content is reached; approximately 9% on a weight basis.

The hydrated inserts are placed into a tightly closed package. The package containing the hydrated inserts is exposed to gamma radiation. The in-vitro dissolution data of the inserts is shown below.

| Radiation Dose | Percent Hydroxypropyl Cellulose Dissolved | | | |
|---|---|---|---|---|
| Range | 2 hr. | 4 hr. | 7 hr. | 23 hr. |
| 0 Mrad | 15 | 39 | 66 | 101 |
| 0.7 to 1.0 Mrad | 18 | 35 | 57 | 101 |
| 1.4 to 1.7 Mrad | 14 | 37 | 47 | 101 |
| 2.2 to 2.5 Mrad | 13 | 25 | 34 | 100 |

WHAT IS CLAIMED IS:

1. An opthalmic insert sterilized by means of gamma radiation comprising hydroxypropyl cellulose and from 0 to 35% by weight medicament which at the time of irradiation consists essentially of a water content of form 6 to 12% by weight.

2. An insert according to Claim 1 which comprises from 0.1 to 35% by weight of medicament.

3. An insert according to Claim 1 which consists essentially of 10% by weight of water.

4. A method of sterilizing an ophthalmic insert comprising hydroxpropyl cellulose and from 0 to 35% by weight of medicament comprising the steps of equilibrating the insert until it consists of from 6 to 12% by weight water, and exposing said equilibrated inserts to a sterilizing dose of radiation.

5. A method according to Claim 4 where said insert consists of 6% by weight water.

6. A method according to Claim 4 where the insert comprises 0.1 to 35% by weight of medicament.

**European Patent Office**

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,X | US-A-4 343 787 (I.M. KATZ)<br>* Example 1; column 5; column 8, line 68; column 9, lines 1-14 *<br>--- | 1-6 | A 61 L 2/08<br>A 61 F 9/00<br>A 61 K 9/06 |
| A | FR-A-2 365 348 (TORAY)<br>* Page 2, lines 8-38 * | 1 | |

-----

|  |
|---|
| TECHNICAL FIELDS SEARCHED (Int. Cl. ³) |
| A 61 L 2/08<br>A 61 K 9/06 |

The present search report has been drawn up for all claims

| Place of search | Date of completion of the search | Examiner |
|---|---|---|
| THE HAGUE | 31-01-1984 | PELTRE CHR. |